# EUROPEAN PATENT APPLICATION

(11) **EP 3 907 193 A1**
(43) Date of publication of application: **10.11.2021**
(21) Application number: 21168158.0
(22) Date of filing: 13.04.2021
(51) Int. Cl.: C02F 1/461, C07H 3/04, C13K 13/00

(54) **METHOD OF PRODUCING LACTULOSE**

(30) Priority: 15.04.2020 RU 2020113569
(71) Applicant: Deep milk procession factory Lactoprom LLC, Aleksin 301360 (RU)
(72) Inventor: YULDASHKIN, Oleg Petrovich, Moscow 109457 (RU)
(74) Representative: Kalkoff & Partner Patentanwälte mbB

(57) **Abstract**

The invention relates to the field of pharmaceutical and food industry, namely to the production of lactulose, which can be used for the treatment and prevention of several diseases, including those reaching gastrointestinal tract. To provide a method of producing lactulose, which leads to a product of high purity without the necessity of a complex and expensive purification and in particular which reduces the usage of ion exchange adsorbent resins for purification, which significantly reduces the costs and/or accelerates the process of production and produces the final product - powdered crystalline lactulose that meets the requirements of the food and pharmaceutical industries, the method of producing lactulose comprises the steps of first preparing an aqueous solution of lactose resulting in a lactose syrup and afterwards electrochemically activating said lactose syrup. The activated lactose syrup is then isomerized by heating the lactose syrup up and holding the heated lactose syrup at an isomerization temperature to obtain a lactose/lactulose syrup, wherein during the heating up and/or during the holding at the isomerization temperature water is added to the lactose syrup to support the isomerization process. Finally, the lactulose is separated from the remaining lactose in the obtained lactose/lactulose syrup and/or the lactulose is purified.

## Description

The invention relates to the field of pharmaceutical and food industry, namely to the production of lactulose, which can be used for the treatment and prevention of several diseases, including those of the gastrointestinal tract.

From the prior art it is already known to produce lactulose by the Lobry de Bruyn-Alberda-Van Ekenstein transformation, that is, by catalyzed transformation of an aldose into the ketose isomer. Accordingly, for this reaction to occur, either at least one catalyst and/or high temperatures over a long reaction time are necessary. However, the use of chemical catalysts such as boric acid or aluminum sulfates is not favored due to the high costs and the complex purification of the produced lactulose to obtain a product for use in the field of pharmaceutical and food industry. Also, the use of high temperatures over a prolonged time has significant disadvantages, in particular a browning of the lactose, leading to numerous undesired side products and accordingly to a low yield as well as the necessity of a cost-intensive purification of the product.

Accordingly, it may be considered an objective of the invention to provide a method of producing lactulose, which leads to a product of high purity without the necessity of a complex and expensive purification and in particular which reduces the usage of ion exchange adsorbent resins for purification.

It may be understood as another objective of the invention to propose a method of producing lactulose that reduces the use of wasteful and/or harmful substances and in particular of chemical catalysts like boric acid, aluminum sulfates etc.

Yet another objective of the invention may be to provide a method of producing lactulose that significantly reduces the costs and/or accelerates the process of production in comparison with all other known methods, and produces the final product, in particular powdered crystalline lactulose that meets the requirements of the food and pharmaceutical industries.

The objective may be achieved by a method of producing lactulose, in particular for use in the field of pharmaceutical and food industry, according to claim 1. Dependent claims refer to preferred embodiments.

The method of producing lactulose, in particular for use in the field of pharmaceutical and food industry, according to the invention comprises the steps of first preparing an aqueous solution of lactose resulting in a lactose syrup and afterwards electrochemically activating said lactose syrup. The activated lactose syrup is then isomerized by heating the lactose syrup up and holding the heated lactose syrup at an isomerization temperature to obtain a lactose/lactulose syrup, wherein during the heating up and/or during the holding at the isomerization temperature water is added to the lactose syrup to support the isomerization process. Finally, the lactulose is separated from the remaining lactose in the obtained lactose/lactulose syrup and/or the lactulose is purified.

The inventors have recognized that using electrochemical activation of the lactose syrup allows the reaction to form lactulose at milder conditions and in particular much faster at high temperature conditions as well as without the use of chemical catalysts like boric acid, aluminum sulfates etc. In particular, the electrochemical activation allows to increase the pH-value of the lactose syrup supporting the isomerization to lactulose. Hence, lactulose can be obtained already with a high purity immediately after the isomerization, wherein as further substances mostly only lactose is present in the syrup after isomerization and the amount of impurities, in particular due to browning is significantly reduced.

The lactulose obtained by the process according to the invention is preferably a powdered crystalline lactulose that meets the requirements of the food and pharmaceutical industries. In particular, the purity of the lactulose is preferably higher than 95 %, more preferably higher than 98 % and most preferably higher than 99 %. Additionally, the lactulose advantageously only contains substances which are considered edible and/or not harmful. Additionally preferred, the obtained lactulose is lactulose trihydrate, especially as dried powder.

For the first step of the method, that is, preparing a lactose syrup, it is preferred that the lactose is solved in water without the addition of other substances in an amount higher than 5 % of the total weight, more preferably not higher than 2 %, most preferably not higher than 0,5 % and in particular no further substances at all are added. The lactose for preparing the lactose syrup advantageously has a purity > 95 %, more advantageously > 99 % and most advantageously > 99.8 %. Also preferably, a pharmaceutical grade lactose and/or lactose monohydrate is used for the preparation of the aqueous solution of lactose. The water used for preparing the lactose syrup is preferably demineralized water or distilled water.

Alternatively, a lower grade lactose might be used and should then be purified first before use for preparing the lactose solution. The purification is preferably done by recrystallization and/or by decanter. Particularly preferred, the lactose is purified by decanter in distilled water. Although a single decanter might be sufficient, a repeated decanter process, in particular two or even better three times, is preferred. For the highest purity, a recrystallization is done after the final decanter. Preferably, the recrystallization is conducted at a temperature of 42°C - 46°C.

The decanter and/or solving the lactose in water is preferably conducted at an elevated temperature, in particular at a temperature between 40°C and 50°C, more preferably between 42°C and 47°C and most preferably between 42°C and 45°C, where the solubility is high, the solving of the lactose is most efficient and at the same time undesired reactions can be avoided.

However, to avoid browning of the lactose syrup, especially due to a melanoidin reaction, the temperature of the lactose syrup and/or of the lactose/lactulose syrup should be lower than 50°C whenever possible, that is, preferably in all steps of the process except the isomerization, which is likely to be conducted at higher temperatures. However, if higher temperatures are necessary than 50°C, the time at higher temperatures has to be kept as short as possible, since at higher temperatures the yield and/or the quality of the lactulose might decrease. On the other hand, the temperature of water to solve the lactose should not be too cold and preferably at least 42°C. Additionally, also at later steps of the process, a temperature above 40°C is favored to keep the solubility as high as possible and to avoid crystallization.

The isomerization according to the invention is induced and/or supported by the previous electrochemical activation of the substances, in particular of the lactose syrup, and additionally by heating the lactose syrup. In particular, the lactose isomerization is supported by adjusting the pH of the lactose syrup, in particular by increasing the pH of the lactose syrup. Accordingly, water is added during the isomerization, which unexpectedly supports the formation of lactulose. Therefore, preferably the water is added during the isomerization when the lactose syrup has already reached the desired isomerization temperature. The lactose/lactulose syrup obtained by the isomerization according to the invention has preferably a high lactulose content of at least 30 %, more preferably of at least 35 %, most preferably of at least 40 % and in particular at least 45%. A much higher lactulose content is not desired since crystallization of the lactulose would occur.

As a final step of the process according to the invention, the lactulose is separated from the remaining lactose in the obtained lactose/lactulose syrup and/or the lactulose is purified. The separation of the lactulose from the lactose and/or other constituents of the lactose/lactulose syrup according to the invention preferably comprises a removal of at least 80 %, more preferably at least 90 % and most preferably at least 95 % of the lactose and/or other constituents. Although a separation or a purification might be enough to obtain a sufficiently pure lactulose, it is advantageous to first separate the lactose from the lactulose and afterwards perform at least one purification step to increase the purity and/or concentration of lactulose in the final product. Accordingly, purification is understood as removing undesired constituents including water from the obtained lactulose or lactulose syrup.

According to a preferred embodiment of the method of producing lactulose, after preparation and/or before electrochemical activation the lactose syrup has a lactose concentration of 21 % - 26 % by weight and preferably the lactose syrup is a saturated solution. Advantageously, the lactose content of the lactose syrup is at least 22 %, preferably between 21 % and 25 %, more preferably between 24 % and 26 % and most preferably 26 %. Generally, any percentages given in this application are percentages by weight if not defined otherwise. To increase the solubility, it might be advantageous to increase the temperature to a range between 40°C and 50°C and preferentially the temperature of the lactose syrup is kept at least at the temperature to avoid crystallization.

To induce and/or support the isomerization, the lactose syrup might first be heated to an isomerization temperature of 93°C - 95°C and/or might be held in the isomerization temperature range, in particular precisely at the isomerization temperature, between 8 and 9 minutes. The isomerization temperature is preferably above 90°C, more preferably between 90°C and 99°C and most preferably between 93°C and 95°C. For the isomerization reaction to occur, the lactose syrup is heated up to the isomerization temperature preferably within a time range between 1 and 20 minutes, more preferably between 1 and 10 minutes and most preferably between 1 and 3 minutes and/or is preferably held at the isomerization temperature between 1 and 20 minutes, more preferably between 5 and 15 minutes, even more preferably between 6 and 10 minutes and most preferably between 8 and 9 minutes. On the one hand, the duration has to be long enough to allow for the isomerization of the majority of the lactose leading to a sufficiently high yield and on the other hand, the duration has to be as short as possible to avoid browning and/or other undesired side reactions. The lactose syrup might be heated up with any heating profile, but a continuous heating is favored. Also advantageously, the isomerization and/or the heating is conducted as a continuous process and/or in a stream of the lactose syrup and not as a batch process. Preferably, the isomerization and/or the cooling is performed in a flow-through pasteurizer and/or cooling unit.

In a preferred embodiment of the method of producing lactulose, the water is added in several, at least in two and in particular in three steps during the heating up and/or during holding the lactose syrup at the isomerization temperature. Additionally preferred, the amount of water added is up to 6 % of the mass of the lactose syrup in total. The water added in several steps might be repeatedly added in a defined portion of water each. When holding the lactose syrup at the isomerization temperature for 8 - 9 minutes, it is especially preferred that water is added after 2, 4 and/or 6 minutes. More generally, it is preferred that the steps of addition of water are approximately equally distributed over the time at the isomerization temperature. Advantageously, the water is only added after the lactose syrup has reached the isomerization temperature or temperature range. Although any amount of water might be added in each step, adding to much water would dilute the lactose syrup and adding to less water would not sufficiently support the isomerization process. Generally, water might be added in total in a range between 1 % and 10 %, preferably between 2 % and 8 % and more preferably between 5 % and 7 % of the starting mass of the lactose syrup.

Most preferably, precisely 6 % of the starting mass of the lactose syrup is added as water during the isomerization. Accordingly, when adding the water in three steps, each portion of water added is 2 % of the starting mass of the lactose syrup. Generally, it is advantageous that in each step the same amount of water is added.

It is especially advantageous that the water added during isomerization has been previously electrochemically activated and/or has been previously heated to the isomerization temperature or to the isomerization temperature range, in particular to 93°C to 95°C before adding to the lactose syrup. Preferably, the electrochemically activated and thereby isomerized water is distilled or demineralized water. Preferably, the water for adding during isomerization has been ionized by an electrochemical activation, preferably to a pH of at least 10, more preferably of at least 11 and most preferably of at least or precisely 12. Alternatively or additionally, the oxidation-reduction-potential (ORP) of the water after the electrochemical activation is preferably between -150 mV and - 300 mV and more preferably between - 180 mV and - 220 mV. Water with such an ORP added to the isomerization process, in particular in a Pasteur, supports the reaction of isomerization of lactose to lactulose at a negative ORP value. That is, the syrup itself during the isomerization reaction at high temperature is kept in the values of negative ORP due to the added electrochemically activated water. The exact parameter of the ORP of a syrup may differ, but it always will be negative. The heating of the water before adding to the isomerization process is preferably conducted in a separate heat exchanger.

In an advantageous embodiment of the method, the temperature of the lactose syrup during the electrochemical activation is below 50°C, preferably between 30°C and 50°C, more preferably between 35°C and 40°C and most preferably between 40°C and 42°C and/or the electrochemical activation of the lactose syrup is conducted until the pH of the activated lactose syrup is above pH 10, preferably above pH 11 and more preferably between pH 11.0 and 12.0. In contrast, the pH-value of the lactose syrup after preparation and before electrochemical activation is typically between pH 4.6 and 5.0, hence the electrochemical activation leads to a significant decrease in acidity and accordingly an increase of the pH-value. Generally, it might also be sufficient to increase the pH to lower values than 11.0 - 12.0, such as 8, 9 or 10, but a higher pH better supports the following isomerization and is therefore preferred.

Since the use of chemical catalysts is not necessary for the isomerization due to the electrochemical activation, it is also preferred that the electrochemical activation of the lactose syrup and/or of the water is conducted without adding chemical agents and/or without adding catalysts to the lactose syrup and/or to the water to keep the lactose syrup and/or the water free of such chemicals. In particular no catalysts are added such as boric acid and/or aluminum sulfates or others. Also, preferably no salts or substances for increasing the conductivity of the lactose syrup or the water are added.

According to a preferential embodiment of the method, the electrochemical activation of the syrup and/or of the water is conducted in a continuous type electrochemical module plant and/or within a processing time between 10 s and 30 s and/or at currents between 5 A - 15 A and voltages between 5 V and 15 V. Preferably, the syrup is treated in an electrochemical activation plant between 2 s and 120 s, more preferably between 5 s and 60 s and most preferably between 10 s and 30 s. The exact time is also dependent on the speed of the feed, i.e. the speed of a flow. Most preferable is a duration of 12 s with a flow rate of 100 liters per hour. Also preferably, the electrochemical module plant is built out of one or several modules, wherein each module is designed to treat from 60 to 100 liters at any given time. Additionally, it is preferred that the modules of an electrochemical module plant can be operated in parallel and/or in series to allow a higher volume processed at once, i.e. the manufacturing capacity and/or a higher flowrate. For example, if the performance has to be 1000 liters per hour, several, in particular ten modules can be used. Additionally, the quantity of modules depends the target pH, with an increasing number of modules for a higher target pH-value. Advantageously, several modules are arranged in series, preferably between 2 and 20, more preferably between 5 and 15 and most preferably 10. Especially preferred, the continuous type electrochemical module plant is a continuous Bakhir's electrochemical module plant and/or the plant consists of continuously gained MB-26 modules.

For the electrochemical activation of the lactose syrup, each module is advantageously being supplied with electricity with a current of 12 A - 16 A, in particular of 14 A - 15 A. Also advantageously, the voltage is between 11 V and 17 V, but most preferable the voltage is between 13 V and 14 V, resulting in an output of a syrup with a pH of 10.8- 12.0, more preferably 11.0 - 11.6, in particular of pH 11.1. For the electrochemical activation of the water, each module, in particular a MV-26 module or a MV-22 module, is advantageously being supplied with electricity with a current of 7 A - 11 A, in particular of 9 A. Also advantageously, the voltage is between 6 V and 10 V, but most preferable the voltage is 7.5 V, resulting in an output of water with a pH of 11 - 12, in particular of pH 11.95.

The electrochemical activation of the syrup and/or of the water might especially be conducted in electrochemical modules having an anode chamber and a cathode chamber, the two chambers being separated by a semipermeable membrane, wherein preferentially the lactose syrup and/or the water passes through the cathode chamber. Although any solution can be circulated through the anode chamber, it is favored that a solution of potassium carbonate (K₂CO₃) in distilled water, in particular with a concentration of 0.6 - 0.8% by weight, is circulated through the anode chamber. This solution only circulates in the anode chamber, but does not contact the syrup due to the semipermeable membrane separating the two chambers. When water is sent to the cathode chamber, it is advantageous to add a small quantity of salt to the water to speed up the ionization process, in particular when using distilled or demineralized water. Preferably, the salt is chemically pure potassium chloride (KCl) and/or the salt is added in concentration of 0.01 - 0.02%, either of which can speed up the ionization process by 1.5 times, if needed.

To avoid unnecessary browning or other undesired reactions, after the isomerization of the lactose syrup and before separation of the lactulose from the obtained lactose/lactulose syrup the obtained lactose/lactulose syrup is preferably cooled to a temperature below 50°C, preferably between 40°C and 50°C, more preferably between 45°C and 50°C and most preferably between 48°C and 50°C. Preferentially, the cooling is conducted as fast as possible to keep the time of the syrup being at a temperature above 50°C as short as possible. Advantageously, the cooling is conducted continuously in flow and/or in a heat exchanger and/or in a pasteurization unit, wherein the cooling agent is preferably ice cold water and not propylene glycol or other low temperature cooling agents, to avoid crystallization, in particular on the interior walls of heat exchanger of the pasteurization unit.

To further process the obtained lactose/lactulose syrup, in a preferred embodiment of the method the obtained lactose/lactulose syrup is neutralized by electrochemical activation, preferably to a target pH-range between 3.0 and 6.0 and more preferably between 3.2 and 5.8 and most preferably between 3.8 and 3.9 after the isomerization of the lactose syrup and/or after cooling. After isomerization, the syrup typically has a pH of 7.1 to 7.5, so during the neutralization the pH has to be lowered. The electrochemical activation is advantageously conducted using an electrochemical module plant, in particular a continuous type electrochemical module plant and most preferably modules identical to the modules used in the previous electrochemical activation steps. For the electrochemical activation to decrease the pH-value, the syrup is advantageously sent to the anode chamber.

For the electrochemical activation of the lactose/lactulose syrup, each module is advantageously being supplied with electricity with a current of 5 A - 11 A, in particular between 7 A and 8 A. Also advantageously, the voltage is between 7 V and 11 V, but most preferable the voltage is between 8 V and 9 V, resulting in an output of a syrup with a pH of 3.2 - 5.8, more preferably 3.7 - 3.9. The temperature during neutralization is preferably between 40°C and 50°C, wherein a recommended operating temperature for the modules when working with syrup is exactly in the range of 42°C to 46°C. However, it might also be advantageous to perform the electrochemical activation in a temperature range between 48°C and 50°C, as obtained after cooling the lactose/lactulose syrup. A solution of any food salt can also be added to the cathode chamber, for example, NaCl or KCl, in particular in a concentration of not more than 0.2%. This solution only circulates in the cathode chamber, but never contacts the syrup itself.

In a further preferred embodiment of the method, for decolorization the lactose/lactulose syrup is purified on at least one ion exchange column, preferably at a temperature of 48°C - 50°C after the isomerization of the lactose syrup and/or after cooling and/or after the neutralization by electrochemical activation. The purification is preferably conducted at an unchanged temperature, in particular between 48°C and 50°C. Advantageously, the decolorization is conducted as a periodic type of process and/or using two or more columns. For the decolorization, the syrup might be treated subsequently in at least two columns and/or several columns might be used in parallel. Using one large continuous column is considered unprofitable, since one large column is expensive in the regeneration process and requires dumping more salt and acid solutions into the sewer, and requires more resins compared to several periodic units. The circulation time at each decolorization column depends on the amount of resin inside, wherein preferably Lewatit S7968 or an analogue resin is used.

Preferentially, the lactose/lactulose syrup is condensed on a vacuum evaporation unit, preferably to a lactose/lactulose content of above 40 % by weight, after the isomerization of the lactose syrup and/or after cooling and/or after the neutralization by electrochemical activation and/or after decolorization. Usually, the content of solids, in particular the content of lactose and lactulose in the syrup before the vacuum evaporation is between 19.8 % and 24.5 % by weight. After the evaporation, the content of lactose and lactulose in the syrup is preferably above 40 %, more preferably, between 40 % and 50 % and most preferably 44 %. The vacuum evaporation or at least one step of the vacuum evaporation is preferably conducted at a temperature below 50°C, preferably between 40°C and 50°C, more preferably between 45°C and 50°C and most preferably between 48°C and 50°C.

The vacuum evaporation unit is preferably a film VE plant and/or a continuous type and/or the evaporation is done by film vacuum evaporation. Advantageously, the vacuum evaporating process is conducted in more than one, preferably in two stages. The first stage has preferably a temperature between 40°C and 70 °V, more preferably between 6o°C and 70°C and most preferably of 65°C and/or preferably the absolute pressure is between 100 mbar and 500 mbar, more preferably between 200 mbar and 300 mbar, most preferably between 225 mbar and 275 mbar and in particular 252 mbar. The second stage has preferably a temperature between 40°C and 50°C, more preferably between 45°C and 50°C, most preferably between 48°C and 50°C and in particular of 50°C and/or preferably the absolute pressure is between 50 mbar and 200 mbar, more preferably between 75 mbar and 150 mbar, most preferably between 100 mbar and 150 mbar and in particular 124 mbar. A higher temperature is not favored, since the syrup is not stable at higher temperatures. It is also advantages to cool the lactose/lactulose syrup down again, preferably as fast as possible, in particular to a temperature between 40°C and 42°C, after the vacuum evaporation.

To separate the lactulose from the obtained lactose/lactulose syrup preferably liquid chromatography is utilized, in particular at a temperature between 40°C and 42°C, obtaining a lactose syrup and a lactulose syrup. The obtained lactose syrup and lactulose syrup are preferably stored in separate tanks for storage and further processing. The lactose syrup obtained by liquid chromatography might be sent to a lactose production room and is preferably not used in lactulose production again.

According to a highly preferred embodiment of the method, the purification of the lactulose from the lactulose syrup is done by film vacuum evaporation to reduce the water content of the lactulose syrup, followed by a long-time crystallization to obtain lactulose crystals and/or by drying the lactulose syrup or the lactulose crystals by method of agglomeration in a fluidized bed, in particular under vacuum. The purification also includes concentration, in particular removal of solvent and/or water. Finally, the dried lactulose, in particular by method of agglomeration, is sent to a package room. Alternatively the lactulose syrup, in particular the lactulose syrup after long term crystallization, is sent to decanter to divide the crystals of lactulose.

Generally, the lactulose syrup is preferably concentrated and/or purified by one of two different and independent processes: The first process is drying the lactulose syrup by method of agglomeration in a fluidized bed, where the carrier is preferentially finely ground and/or fine-disperse lactulose, in particular lactulose trihydrate. Especially preferred the fine-disperse lactulose has a crystal size of less than 100 µm, more preferably less than 50 µm and most preferably less than 40 µm. Alternatively the other process is, evaporating parts of the solvent, in particular the water, by film vacuum evaporation, where preferentially the same vacuum evaporation unit and/or the same parameters as in the previously conducted vacuum evaporation are used. Advantageously, the solvent is removed until the content of the lactulose is at least 60 %, more preferably 75 %, most preferably 80 % and in particular 82 % by weight.

The long-time crystallization is preferentially conducted in a special crystallization unit, in particular in a special buffer tank and/or with periodic cooling and stirrer cycles. The long-term crystallization is performed over at least 24 h, preferably over at least 3 days, more preferably at least 5 days and most preferably at least 7 days, in particular exactly or not more than 7 days. After crystallization, the separation of the formed crystals is performed in one or several centrifuges. Afterwards, the separated crystals are advantageously dried under vacuum in a fluidized bed, resulting in dried lactulose trihydrate.

These and other aspects of the invention will be apparent from and elucidated with reference to the detailed embodiment described hereinafter.

The method of production of lactulose includes preparation of a solution of pharmaceutical grade lactose, that is, lactose monohydrate 99.8 % purity, in distilled water with a concentration of 26 %.

The second step of the method is an electrochemical activation of the obtained lactose syrup on a continuous electrochemical module plant at a temperature of 40-42°C until reaching a pH-value of the syrup between 11 and 12. Afterwards, for the isomerization of the lactose syrup the syrup is continuously heated to 95°C and maintaining in a stream at this temperature for 8-9 minutes. Isomerized distilled water is subsequently added during the exposure to the high temperature on the 2nd, 4th and 6th minute.

The added distilled water is previously ionized to a pH-value of 12 and an oxidation-reduction potential (ORP) not smaller than -220 mV and not higher than -300 mV. The preparation of the added ionized or rather isomerized water is performed on Bahir MB-26 modules, which are modules of a continuous type. Each module has two independent chambers, an anode and cathode, separated by a semipermeable membrane. A solution of potassium carbonate (K₂CO₃) in distilled water with a concentration of 0.6 - 0.8% is sent to the anode chamber, only circulating in the anode chamber, but not contacting the lactose syrup in any way, due to the separation by the membrane between both chambers. The distilled water to be isomerized is sent to the cathode chamber. Preferentially, chemically pure potassium chloride (KCl) with a concentration of 0.01 % - 0.02 % is added to the distilled water to speed up the ionization process of distilled water by 1.5 times. Before adding the water to the isomerization process of the lactose syrup, the water is separately heated to the same temperature of 95°C utilizing a heat exchanger.

This water is added in the total amount of not more than 6% of the mass of the lactose syrup. The water is added at the 2^{nd}, 4^{th} and 6^{th} minute, respectively, by equal amounts of 2 % calculated from the starting lactose syrup mass.

The lactose/lactulose syrup after the isomerization and continuous heating is being cooled again to 48°C - 50°C in a pasteurization unit as quickly as possible, wherein the cooling agent needs to be ice cold water, not propylene glycol or other - to avoid crystallization on the interior walls of the heat exchanger.

After cooling, the lactose/lactulose syrup is neutralized in a continuous Bakhir's electrochemical module plant, where pH of syrup becomes 3.8 - 6.0 at the same temperature of 48°C - 50°C. Then the resulting syrup with temperature still 48°C - 50°C is purified on ion exchange columns for means of decolorization. The decolorization is a periodic type of process, where preferably two or more columns are used in parallel. The circulation time at each decolorization column depends on the amount of resin inside. Preferentially for the ion exchange column, Lewatit S7968 or analogue resin is used. Additionally, the temperature of this process is not changed.

After decolorization, the lactose/lactulose syrup is condensed on a vacuum evaporation unit, in particular a film VE plant of a continuous type. The starting syrup is 19.8 % - 24.5 % and it evaporates to 44 % of T.S. After the evaporation process, the lactose/lactulose syrup is chilled again to 40 - 42°C. Afterwards, the lactose/lactulose syrup is separated on liquid chromatography units at the same temperature of 40°C - 42°C, and the two separated syrups, the lactose and the lactulose syrup, go to different buffer tanks.

The lactulose syrup is sent to one of two different and independent processes:
Either the lactulose syrup is dried by method of agglomeration in a fluidized bed, where the carrier is finely ground and/or fine-disperse lactulose, in particular lactulose trihydrate, with a crystal size of not more than 40 µm.

Or alternatively, the lactulose syrup is evaporated in a before mentioned film vacuum evaporation unit till 82% T.S. and afterwards a long-time crystallization is performed in a special crystallization unit, which is a special buffer tank with its own periodic system of cooling and stirring. This process of crystallization can last up to 7 days. Separation of the formed crystals is finally done in centrifuges, after which the obtained crystals are dried under vacuum in a fluidized bed.

The lactose syrup obtained by the liquid chromatography is sent to a lactose production room and cannot be used in lactulose production ever again. Also, the dried lactulose is sent to the package room.

## Claims

1. Method of producing lactulose, in particular for use in the field of pharmaceutical and food industry, with the steps
- preparation an aqueous solution of lactose to produce a lactose syrup;
- electrochemical activation of the lactose syrup;
- isomerization of the lactose syrup by heating the lactose syrup and holding the heated lactose syrup at an isomerization temperature to obtain a lactose/lactulose syrup, wherein
- during the heating and/or during the holding at the isomerization temperature water is added to the lactose syrup; and
- separation of the lactulose from the obtained lactose/lactulose syrup and/or purification of the lactulose.

2. Method of producing lactulose according to claim 1, **characterized in that** the lactose syrup has a lactose concentration of 21 % - 26 % by weight.

3. Method of producing lactulose according to claim 1 or 2, **characterized in that** in the step of isomerization the lactose syrup is first heated to an isomerization temperature of 93°C - 95°C and/or held in the isomerization temperature range between 8 and 9 minutes.

4. Method of producing lactulose according to at least one of the proceeding claims, **characterized in that** the water is added in several steps during the heating up and/or during holding the lactose syrup at the isomerization temperature and/or that the amount of water added is up to 6 % of the mass of the lactose syrup in total.

5. Method of producing lactulose according to at least one of the proceeding claims, **characterized in that** the water added during isomerization has been previously electrochemically activated and/or has been previously heated to the isomerization temperature before adding to the lactose syrup.

6. Method of producing lactulose according to at least one of the proceeding claims, **characterized in that** the temperature of the lactose syrup during the electrochemical activation is between 40°C and 42°C and/or the electrochemical activation of the lactose syrup is conducted until the pH of the activated lactose syrup is between pH 11.0 and 12.0.

7. Method of producing lactulose according to at least one of the proceeding claims, **characterized in that** the electrochemical activation of the lactose syrup and/or of the water is conducted without adding chemical agents and/or without adding catalysts to the lactose syrup and/or to the water.

8. Method of producing lactulose according to at least one of the proceeding claims, **characterized in that** the electrochemical activation of the syrup and/or of the water is conducted in a continuous type electrochemical module plant and/or within a processing time between 10 s and 30 s and/or at currents between 5 A - 15 A and voltages between 5 V and 15 V.

9. Method of producing lactulose according to at least one of the proceeding claims, **characterized in that** the electrochemical activation of the syrup and/or of the water is conducted in electrochemical modules having an anode chamber and a cathode chamber, the two chambers being separated by a semipermeable membrane, wherein the lactose syrup and/or the water passes through the cathode chamber.

10. Method of producing lactulose according to at least one of the proceeding claims, **characterized in that** after the isomerization of the lactose syrup the obtained lactose/lactulose syrup is cooled to a temperature between 48°C and 50°C.

11. Method of producing lactulose according to at least one of the proceeding claims, **characterized in that** after the isomerization of the lactose syrup and/or after cooling, the obtained lactose/lactulose syrup is neutralized by electrochemical activation to a target pH-range between 3.2 and 5.8.

12. Method of producing lactulose according to at least one of the proceeding claims, **characterized in that** after the isomerization of the lactose syrup and/or after cooling and/or after the neutralization by electrochemical activation, the lactose/lactulose syrup is purified on at least one ion exchange column for decolorization at a temperature of 48°C - 50°C.

13. Method of producing lactulose according to at least one of the proceeding claims, **characterized in that** after the isomerization of the lactose syrup and/or after cooling and/or after the neutralization by electrochemical activation and/or after decolorization, the lactose/lactulose syrup is condensed on a vacuum evaporation unit to a lactose/lactulose content of above 40 % by weight.

14. Method of producing lactulose according to at least one of the proceeding claims, **characterized in that** separation of the lactulose from the obtained lactose/lactulose syrup is achieved by liquid chromatography at a temperature between 40°C and 42°C obtaining a lactose syrup and a lactulose syrup.

15. Method of producing lactulose according to at least one of the proceeding claims, **characterized in that** the purification of the lactulose from the lactulose syrup is done by film vacuum evaporation to reduce the water content of the lactulose syrup, followed by a long-time crystallization to obtain lactulose crystals and/or by drying the lactulose syrup or the lactulose crystals by method of agglomeration in a fluidized bed, in particular under vacuum.
